# EUROPEAN PATENT SPECIFICATION

(11) **EP 1 424 978 B1**
(45) Date of publication and mention of the grant of the patent: **21.07.2010**
(21) Application number: 02770355.2
(22) Date of filing: 06.09.2002
(51) Int. Cl.: A61F 13/15

(54) **A METHOD OF PRODUCING A FIBROUS MATERIAL LAYER, A FIBROUS MATERIAL LAYER AND AN ABSORBENT ARTICLE CONTAINING SAME**
VERFAHREN ZUR HERSTELLUNG EINER FASERIGEN MATERIALSCHICHT, EINE FASERIGE MATERIALSCHICHT UND EIN DIESE ENTHALTENDER SAUGFÄHIGER ARTIKEL
PROCEDE DE PRODUCTION D'UNE COUCHE EN MATIERE FIBREUSE, COUCHE DE MATIERE FIBREUSE ET ARTICLE ABSORBANT CONTENANT CELLE-CI

(30) Priority: 06.09.2001 SE 0102973
(43) Date of publication of application: 09.06.2004
(73) Proprietor: SCA Hygiene Products AB, 405 03 Göteborg (SE)
(72) Inventor: WAHLSTRÖM, Johan, S-412 68 Göteborg (SE); WÄSTLUND-KARLSSON, Jan, S-431 49 Mölndal (SE)
(74) Representative: Egeröd, Lisbeth
(86) International application number: PCT/SE2002/001614
(87) International publication number: WO 2003/020192

(56) References cited:
- EP-A1- 0 937 792
- WO-A1-99/30661
- US-A- 4 360 022
- US-A- 4 798 603
- US-A- 5 382 245
- US-A- 5 669 895
- US-A- 6 093 474

## Description

### Technical field

The present invention relates to a method of producing a fibrous material layer mainly intended for being incorporated in an absorbent article, such as a diaper, pant diaper, incontinence guard, sanitary napkin, wound dressing and the like. It further refers to a fibrous material layer and an absorbent article containing same.

### Background of the invention

Absorbent articles of the above-mentioned kind are intended for absorption of body fluids, such as urine and blood. As a liquid pervious topsheet, facing the wearer during use, they usually exhibit a nonwoven material, for example of spunbond-type. It is also previously known to arrange a liquid acquisition layer between the top layer and the absorbent body, said liquid acquisition layer having the ability to quickly receive large quantities of liquid, and to distribute the liquid and temporarily store it before it is absorbed by the underlying absorbent body. This is of great importance, especially in the thin compressed absorbent bodies of today, often comprising a high content of so called superabsorbents, which certainly have a high absorption capacity but in many cases a too low absorption rate in order to momentously be able to absorb the large quantity of liquid which can be discharged within a few seconds during urination. A porous, relatively thick acquisition layer, for example in the form of a fibrous wadding, a carded fibrous web, or another type of fibrous material, has a high instantaneous liquid-receiving capacity and is able to store the liquid temporarily until it has been absorbed by the absorbent body. The same applies for porous foam materials. The liquid is thereafter drained successively into the adjacent absorbent body, after which the acquisition layer once again has the capacity to receive liquid from a repeated wetting.

Examples of absorbent articles comprising such porous acquisition layers are, for example, disclosed in US-A-3,371,667, EP-A-0,312,118 and EP-A-0,474,777.

The materials used today as acquisition layers in absorbent articles are mostly functioning well, but are relatively expensive and can sometimes exhibit an insufficient acquisition rate, especially in the second and third wettings, if large quantities of liquid are involved. Furthermore, they are difficult to process and store due to their bulkiness.

It is previously known through EP-A-0,391,814 and GB-B-2,209,672 to use continuous, unbonded synthetic fibres, so-called tow, in absorbent articles for distributing liquid in the longitudinal direction of the article.

It is further known through WO 99/27876 to provide a tow layer used in an absorbent article as an acquisition and/or topsheet material and which has been bonded in points, lines or spots in a bonding pattern, but where the tow filaments otherwise are substantially unbonded to each other. This material layer exhibits a high acquisition rate for liquid also when repeatedly wetted, exhibits a high strength in longitudinal direction, high resistance and high comfort. One problem that may occur with this type of material is that it is difficult to achieve a high strength in the transverse direction, since said strength being provided solely by the bonding pattern.

### Object and most important features of the invention

The object of the present invention is to provide a method of producing a tow layer of the above kind, which exhibits an improved strength especially in the transverse direction. According to the invention, this has been achieved by the fact that bonding is performed in two steps: a first step wherein bonding takes place by thermobonding, such as ultrasonic welding or laser welding, at which a pattern roll is used which provides the desired bonding pattern, said pattern roll being driven at a higher speed than the feeding speed of the tow layer, so as to create a relatively diffuse and strong bonding pattern in the tow layer; and a second step in which a more distinct second bonding pattern is created.

Preferably that said first and second bonding patterns are applied so as to overlap with each other over at least a substantial part of the bonded area of said fibrous material layer. A substantial portion in this respect means at least 50% of the bonded area of the fibrous layer.

It is further preferred that that also in the second step bonding takes place by thermobonding, such as ultrasonic welding or laser welding, at which a pattern roll is used which provides the desired bonding pattern, wherein the second bonding step the pattern roll is driven at substantially the same speed as the feeding speed of the tow layer.

In a preferred embodiment the tow layer is in the second step laminated to a web of material, such as a nonwoven layer, with said second bonding pattern.

The invention further refers to a fibrous material layer mainly intended for being incorporated in an absorbent article, said material layer comprises a layer of continuous fibres, so-called tow, which have been bonded together in points, lines or spots in a bonding pattern, but otherwise are substantially unbonded to each other,
wherein the tow layer is bonded with at least two bonding patterns:
a first relatively strong and diffuse bonding pattern provided by thermobonding, and a second more distinct bonding pattern;
said first and second bonding patterns are arranged so as to overlap with each other over at least a substantial part of the bonded area of said fibrous layer.

The invention also refers to an absorbent article such as a diaper, pant diaper, incontinence guard, sanitary napkin, wound dressing and the like, of the kind comprising a liquid permeable topsheet, a liquid impervious backsheet and an absorbent body arranged therebetween, wherein the article comprises a tow layer of the kind stated above.

The layer can be used as a liquid acquisition layer underneath a topsheet, as a topsheet, or as an integrated topsheet/liquid acquisition layer.

Further features of the invention are evident from the following description and the claims.

### Description of the drawings

The invention will below be closer described with reference to some of the embodiments shown in the accompanying drawings.
Figure 1 is a plan view of an absorbent article in the form of an incontinence guard.
Figure 2 is a section according to the line II-II in Figure 1.
Figure 3 shows schematically a piece of a fibrous material layer according to the invention.
Figure 4 is a schematic side view of a process equipment for performing the method according to the invention.

### Description of embodiments

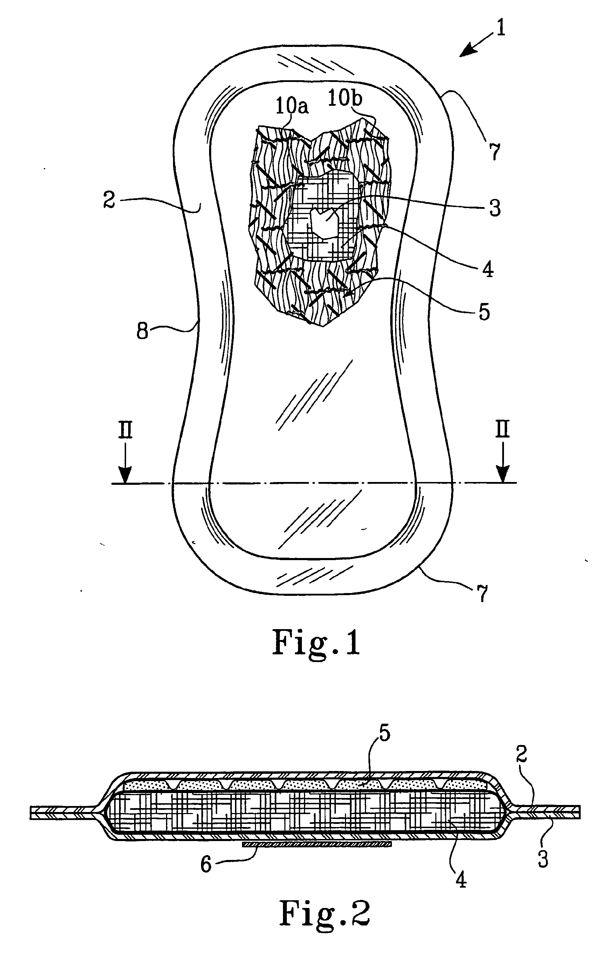

Figure 1 and 2 show schematically an example of an incontinence guard 1 comprising a liquid pervious topsheet 2, a liquid impervious backsheet 3 and a absorbent body 4 enclosed therebetween. A porous resilient liquid acquisition layer 5 is arranged between the liquid pervious topsheet 2 and the absorbent body 4.

The liquid pervious topsheet 2 can comprise a nonwoven material, for example a spunbond material of synthetic filaments, a meltblown material, a thermobonded material or a bonded carded fibrous material. The liquid impervious backsheet 3 can consist of a plastic film, a nonwoven material which is coated with a liquid impervious material or a hydrophobic nonwoven material which resists liquid penetration.

The topsheet 2 and the backsheet 3 have a larger surface area than the absorbent body 4 and the liquid acquisition layer 3 and extend outside the edges thereof. The layers 2 and 3 are interconnected within the projecting portions, for example by gluing or welding with heat or ultrasonic.

The absorbent body 4 can be of any conventional kind. Examples of common absorption materials are cellulosic fluff pulp, tissue layers, highly absorbent polymers (so called superabsorbents), absorbent foam materials, absorbent nonwoven materials and the like. It is common to combine cellulosic fluff pulp with superabsorbents in an absorbent body. It is also common to have absorbent bodies comprising layers of different materials with different properties concerning liquid acquisition capacity, liquid distribution capacity and liquid storage capacity. This is wellknown for the person skilled in the art and need not be described in detail. The thin absorbent bodies which are common in for example baby diapers and incontinence guards often consist of a compressed mixed or layered structure of cellulosic fluff pulp and superabsorbent.

On the outside of the liquid impervious backsheet 3 fastening means in the form of strips 6 of a selfadhesive glue are arranged. An incontinence guard of the kind shown in Figure 1 is mainly intended to be used by persons suffering from a relatively light incontinence and is easily worn in ordinary underpants. The fastening means 6 serve to keep the incontinence guard in place in the underpants during use. A number of other types of glue patterns, for example transverse, are of course possible as well as other types of fastening means such as hook and loop, snap fasteners, girdles, special under-pants or the like.

The incontinence guard is hour glass shaped with broader end portions 7 and a more narrow crotch portion 8 located between the end portions. The crotch portion 8 is the portion of the incontinence guard that is intended during use to be worn in the crotch between the legs of the wearer and serve as a receiving portion for the discharged body fluid.

It should be noted that the incontinence guard shown in the drawings and described above only is a non-limiting example of an absorbent article. Thus the shape of the article as well as the construction thereof can be varied. The absorbent article can also be a diaper, a pant diaper, a sanitary napkin or the like. The absorbent article can be disposable or reuseable. For reuseable articles other materials than the above described are however used as a liquid pervious topsheet and absorbent body respectively.

Between the liquid pervious topsheet 2 and the absorbent body 4 there is arranged a porous and resilient acquisition layer 5 having the ability to quickly receive large amounts of liquid and distribute the liquid and store it temporarily before it is absorbed by the underlying absorbent body 4. This ability should be essentially maintained also after wetting of the material. The acquisition layer 5 can either cover the entire absorbent body 4, extend outside thereof or cover only part of the central portions of the absorbent body.

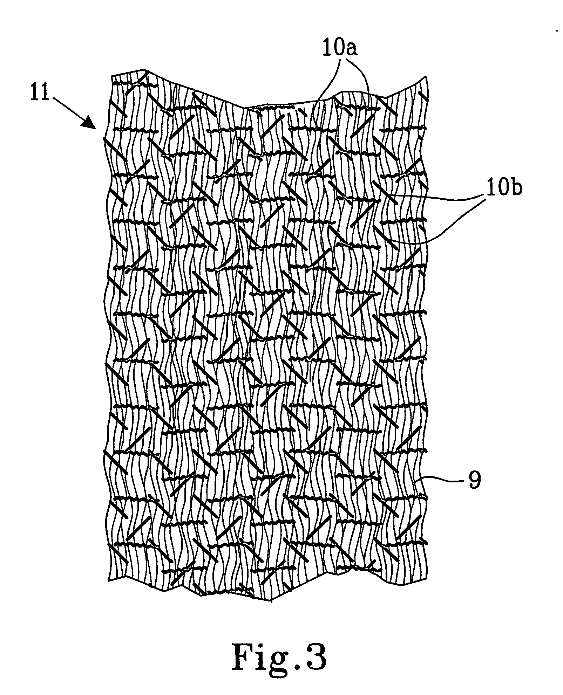

According to the invention the acquisition layer 5 consists of a layer of continuous filaments or fibers 9, so called tow fibers, which have been bonded together in points, spots or lines forming a bonding pattern 10, but otherwise are substantially unbonded to each other. In the embodiment shown in Figure 1 and 3 the bonding pattern 10 is a pattern of lines with short lines arranged in a zigzag configuration. The bonding pattern is achieved by for example ultra sonic welding or other thermal bonding. Examples of other suitable thermal bonding methods are pattern calendering, laser bonding etc. This implies that at least some of the fibers in the tow are thermoplastic. Examples of thermo-plastic fibers are polyolefines, polyamides, polyester and the like. Also so called bicomponent fibers are included. The choice of bonding type is mainly decided by which type of fibers are used in the tow.

The design of the bonding pattern 10 can of course vary within wide limits. The pattern may be in the form of points, spots or preferably lines. The lines may be straight as well as curved and the length can vary from a few millimeters to extending transversely or diagonally across the entire article. Preferably the lines extend across or obliquely across the longitudinal direction of the tow fibers 9, so that a plurality of tow fibers are bonded to each other by each bonding line. It is also an advantage if different bonding lines overlap each other as seen across the longitudinal direction of the tow fibers, so that a main part of the tow fibers are bonded at least at some part of their length. The tow fibers 9 are except at the bonding sites unbonded to each other.

The bonding pattern can be the same over the entire acquisition layer 5 or be different in different parts thereof, thus the bonding pattern can be more sparse in the wetting area and tighter outside thereof. It is also possible to design the bonding pattern in such a way that the layer 5 will have different thickness in different parts of the article, for example thinner in the central portions thereof and thicker in the surrounding edge portions in order to create a bowl shape which provides a liquid receiving volume, alternatively thicker in the central portions than in the surrounding edge portions in order to provide a better body contact.

The layer 15 of tow fibers 9 according to the invention can, besides as a liquid acquisition layer in an absorbent article, be arranged as a topsheet material closest to the wearer or as a combined topsheet/acquisition layer. It can also be bonded to a carrier material, for example a nonwoven.

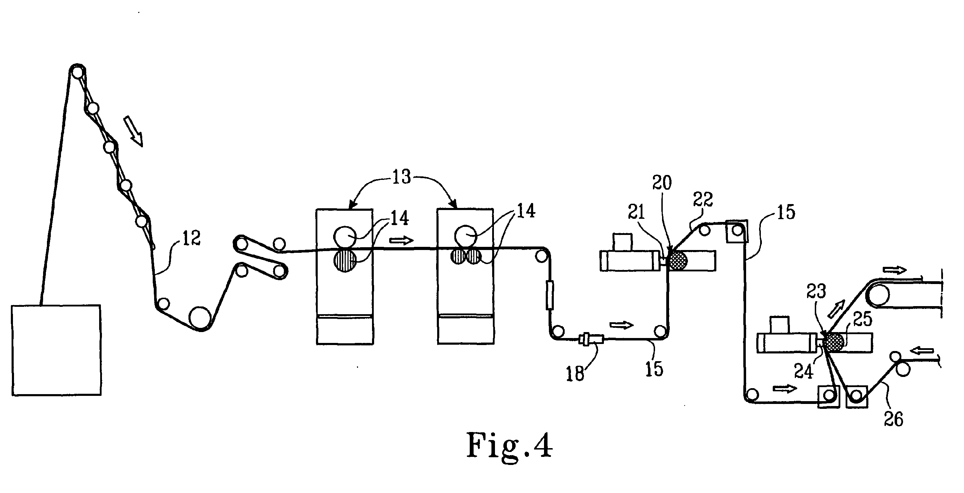

The method of producing the material layer according to the invention comprises several steps, which are schematically illustrated in Figure 4. Fiber tow 12 is supplied in sacks or in the form of bales or rolls of continuous tow fibers, which either are straight, crimped or curled. Crimped or curled fibers are preferred in this case since they provide a very open and airy structure. The fibers in the tow can be of any suitable material such as polyethylene, polypropylene, polyamide, polyester, polylactide, polyvinyl acetate, cellulose acetate, regenerated cellulose such as viscose and rayon, or of bicomponent type with a shell of a polymer having a lower melting point and a core of a polymer having a higher melting point. Specially preferred are such fibers having a high resiliency, for example polyester, copolyester and polypropylene.

The fiber thickness can vary but should be in the interval 0.5 to 10 dtex, preferably 1.5 to 25 and most preferably 2-15 dtex, if the material is to be used as an acquisition layer. The open airy structure in combination with the relatively coarse fiber dimension gives a very rapid liquid acquisition. Besides the material is strong due to the continuous fibers which provide strength in the longitudinal direction, and the bonding pattern which provides strength in the transverse direction.

The bales or the like are opened in special opening equipments in which the tow fibers are separated from each other, stretched and spread out to an essentially evenly thick layer of tow fibers. The tow layer is bonded in the desired bonding pattern according to above and is cut in suitable lenghts either before or after application in an absorbent article. The bonding can alternatively be made after cutting. A tow is a relativley cheap delivery form of fibers as compared to nonwoven, waddings or the like which are normally used as acquisition materials.

As can be seen from Figure 4 the opening device 13 comprises one or more pairs of rolls forming a roll nip, said rolls either being smooth or one roll is threaded and the opposite roll is a counter roll, the fiber tow being fed through said roll nip(s), which provides a separation of the fibers. The tow fibers are stretched during their passage through the roll nip(s) 14. This type of opening devices are of conventional kind and are available on the market in different constructions.

According to the embodiment shown in Figure 4 the opened fiber tow, which now is in the form of a spread-out layer of separated individual tow fibers 9, is led through an ejector 18 which blows air into the material web 15 substantially in the longitudinal direction thereof. This ejector and through-air blowing is optional, depending on the quality of the fiber tow, and is used for increasing volume and bulkiness of the material web. The device may comprise further means for controlling the fiber distribution in the transverse direction before entering the bonding station 20, such as folding means for longitudinally folding the tow layer one or more times and/or guiding means having a certain cross sectional shape through which the tow layer 15 is fed. A more detailed description of a method and device for making a bonded tow layer is found in EP-A-937 792, the content of which is incorporated herein by reference.

The tow layer 15 is then fed to a first bonding station 20, which in this embodiment is an ultra sonic welding device. This comprises an ultra sonic horn 21 arranged just opposite a pattern roll 22.

The pattern roll 22 can besides a macropattern, e g a pattern of lines or other optional pattern, be provided with a micropattern, in the form of an uneven or grooved surface, on the top surface of the protruded parts of the pattern roll 22. By the micropattern the total welding surface is divided in smaller parts, at which there will be less material to melt and the friction between the material web and the ultrasonic welding device is reduced. The height of the micropattern is of the magnitude tenth parts of a millimeter, this applies also for their length and width. The micropatten makes it possible to weld at higher speeds without risk that the joint breaks during bonding. It would also be possible to weld an uneven material web, i e in which the material thickness varies across the machine direction.

It would also be possible to have an uneven or grooved surface on the ultrasonic horn 21.

In the first bonding station 20 a relatively strong and diffuse first bonding pattern 10a is created. This is achieved by the fact that the pattern roll 22 is driven at a somewhat higher speed than the feeding speed of the tow layer 15. The speed of the pattern roll 22 should be at least 8%, preferably between 8 and 100% and more preferably between 10 and 25% higher than the feeding speed of the tow layer 15. The speed difference leads to that the welding points gets diffuse but relatively stronger than bonding points created by driving the pattern roll 22 at the same speed as the feeding speed of the tow layer 15.

As is mentioned above other types of thermal bonding methods than ultrasonic welding can be used, such as pattern calendering, laser bonding etc. The second bonding pattern may also be provided by other bonding techniques than thermal bonding, such as by gluing or mechanical bonding.

The tow layer 15 is after the first bonding station 20 fed to a second bonding station 23, which in this embodiment also is an ultra sonic welding device. This comprises an ultrasonic horn 24 arranged just opposite a pattern roll 25. In this second bonding station 23 the pattern roll 25 is driven at substantially the same speed as the feeding speed of the tow layer15. The bonding pattern 10b formed in the tow layer15 will then be relatively distinct. Since it is applied on top of the first bonding pattern 10a it will be the most visible of the two bonding patterns.

The first and second bonding patterns are applied so as to be overlapping over at least a substantial part, meaning at least 50%, of the bonded area of the tow layer. The term overlapping does not necessarily mean that the individual bonding sites have to overlap or cross each other, but that the bonding patterns are overlapping.

There are several advantages achieved by the present invention. Firstly the strength in transverse direction of the bonded tow layer is considerably increased by the first relatively diffuse bonding pattern. An increase of at least 50% of the transverse strength has been achieved as compared to the strength obtained by a distinct bonding pattern provided by driving the pattern roll with the same speed as the tow layer. A further advantage is that the tow layer is evened in the first bonding station, so that it when entering the second bonding station is more even than it would have been otherwise. The second bonding is therefore improved with a reduced risk for loose fibers sticking out.

In the embodiments shown in the drawings the first and second bonding patterns 10a and 10b are different with the second bonding pattern 10b being more dense than the first more diffuse bonding pattern 10a. It is of course possible to have a more dense first bonding pattern and also to have the same first and second bonding patterns

Bonding density or tightness, is defined as the number of bonding sites per area unit.

The bonding pattern comprises a plurality of bonding sites in the form of points, lines, spots or the like arranged in a pattern. The bonding area of a bonding pattern is defined as the amount of the pattern that consists of the bonding sites. The first and second bonding patterns may have substantially the same or different bonding areas.

It is pointed out that by relatively large bonding sites, for example in the form of lines, a relatively large bonding area may be provided with a relatively small number of bonding sites, as compared to a bonding pattern of small bonding sites, for example in the form of points, which have to arranged tighter in order to provide the same bonding area as for a pattern of larger bonding size. Thus both bonding area and bonding tightness are important.

The bonding sites, when these have a length extension in one main direction, such as lines, of the first and second bonding patterns may further have the same or different angles with respect to the main direction of the tow fibers 9, so that for example the bonding lines or the like of the first bonding pattern are arranged more close to a perpendicular direction with respect to the main direction of the tow fibers as compraed to the bonding lines of the second bonding pattern or vice versa. By this an increase of the bonding strength in the transverse direction of the layer is achieved. The bonding lines within the first and second bonding patterns may also have different angles with respect to the main direction of the tow fibers.

In this second bonding station 23 the tow layer can possibly be laminated to a nonwoven material 26 or a plastic film, which may be perforated or breathable. The nonwoven material 26 or the like can either be laminated to the tow layer 15 over the entire width thereof or in the form of strips be laminated only to the edges of the material web. The nonwoven material 26 or the like, which is either hydrophobic or hydrophilic, serves to prevent spreading of liquid toward the edges of the absorbent article and to prevent rewetting of liquid towards the skin of the wearer.

The pattern bonded tow layer15, which optionally has been laminated to a nonwoven material or the like, can then either be wound on a winding roll or directly fed into a diaper machine or the like, where it is applied as a layer in an absorbent article such as a diaper, a pant diaper, an incontinence guard, a sanitary napkin or the like.

## Claims

1. A method of producing a fibrous material layer mainly intended for being incorporated in an absorbent article, such as a diaper, pant diaper, incontinence guard, sanitary napkin, wound dressing and the like, comprising opening at least one bundle of continuous fibers, so called tow , separating the tow fibers and evening the tow to a layer (15) having the desired fiber distribution and bonding the tow layer in a bonding pattern (10), but where the tow fibers otherwise are substantially unbonded to each other,
**characterized in that** bonding is performed in two steps:
a first step wherein bonding takes place by thermobonding, such as ultrasonic welding or laser welding, at which a pattern roll (22) is used which provides the desired bonding pattern, said pattern roll (22) being driven at a higher speed than the feeding speed of the tow layer (15), so as to create a relatively diffuse and strong bonding pattern (10a) in the tow layer (15);
and a second step in which a more distinct second bonding pattern (10b) is created.

2. A method as claimed in claim 1,
**characterized in that** said first and second bonding patterns (10a, 10b) are applied so as to overlap with each other over at least a substantial part of the bonded area of said fibrous material layer.

3. A method as claimed in claim 1 or 2,
**characterized in that** also in the second step bonding takes place by thermobonding, such as ultrasonic welding or laser welding, at which a pattern roll (25) is used which provides the desired bonding pattern.

4. A method as claimed in claim 3,
**characterized in that** in the second bonding step the pattern roll (25) is driven at substantially the same speed as the feeding speed of the tow layer (15).

5. A method as claimed in claim 3 or 4,
**characterized in that** the speed of the pattern roll (22) of the first bonding step is at least 8% higher than the feeding speed of the tow layer (15).

6. A method as claimed in claim 5,
**characterized in that** the speed of the pattern roll (22) of the first bonding step is between 8 and 100% and preferably between 10 and 25% higher than the feeding speed of the tow layer (15).

7. A method as claimed in any of claims 1-6,
**characterized in that** in the second step the tow layer (15) is laminated to web of material, such as a nonwoven layer (26).

8. A fibrous material layer mainly intended for being incorporated in an absorbent article, such as a diaper, pant diaper, incontinence guard, sanitary napkin, wound dressing and the like, said material layer comprises a layer (15) of continuous fibres (9), so-called tow, which have been bonded together in points, lines or spots in a bonding pattern (10), but otherwise are substantially unbonded to each other, **characterized in that** the tow layer (15) is bonded with at least two bonding patterns:
a first relatively strong and diffuse bonding pattern (10a) provided by thermobonding, and
a second more distinct bonding pattern (10b);
said first and second bonding patterns are arranged so as to overlap with each other over at least a substantial part of the bonded area of said fibrous layer.

9. A fibrous material layer as claimed in claim 7,
**characterized in that** also in the second bonding pattern (10b) is a thermobonded bonding pattern.

10. A fibrous material layer as claimed in claims 8 or 9,
**characterized in that** a web of material, such as a nowoven layer (26) is laminated to the tow layer (15) with said second bonding pattern (10b).

11. A fibrous layer as claimed in any of claims 8-10,
**characterized in that** at least a part of the continuous fibres (9) in said tow layer are crimped or curled.

12. A fibrous layer as claimed in any one of claims 8-11,
**characterized in that** the first and second bonding patterns (10a,10b) comprise dots, spots or lines which cross the longitudinal direction of the tow fibers (9).

13. A fibrous layer as claimed in claim 12,
**characterized in that** different bonding sites overlap each other, as seen in the transverse direction of the article, so that a main portion of the tow fibers (9) are bonded at least at some part of their length.

14. A fibrous layer as claimed in claim 12 or 13,
**characterized in that** individual bonding sites of the first and second bonding patterns (10a, 10b) have a length extension in one main direction, such as lines or the like, and that the bonding lines or the like of the first bonding pattern (10a) are arranged at a different angle to the main direction of the tow fibers (9) as compared to the bonding lines or the like of the second bonding pattern (10b).

15. A fibrous layer as claimed in any of claims 12-14,
**characterized in that** individual bonding sites of the first and second bonding patterns (10a, 10b) have a length extension in one main direction, such as lines or the like, and that the bonding lines or the like within the first and/or the second bonding pattern have different angles with respect to the main direction of the fibers.

16. An absorbent article, such as a diaper, pant diaper, incontinence guard, sanitary napkin, wound dressing and the like, of the kind comprising a liquid permeable topsheet (2), a liquid impervious backsheet (3) and an absorbent body (4) arranged therebetween,
**characterized in that** the article comprises a tow layer as claimed in any of claims 8-15

17. An absorbent article according to claim 16,
**characterized in that** the tow layer is used as a liquid acquisition layer (5) applied between the topsheet (2) and the absorbent body (4).

18. An absorbent article according to claim 16,
**characterized in that** the tow layer is used as a liquid pervious topsheet.

19. An absorbent article according to any one of claims 16-18, **characterized in that** the tow layer is used as an integrated topsheet/liquid acquisition layer.

## Patentansprüche

1. Verfahren zum Herstellen einer Fasermateriallage, die hauptsächlich dafür gedacht ist, in einen Absorptionsartikel, wie zum Beispiel eine Windel, eine Höschenwindel, einen Inkontinenzschutz, eine Binde, einen Wundverband und dergleichen eingebracht zu werden, umfassend ein Öffnen zumindest eines Bündels durchgehender Fasern, eines so genannten Wergs, Trennen der Wergfasern und Glätten des Wergs in eine Lage (15) mit der gewünschten Faserverteilung und Binden der Werglage in ein Bindemuster (10), aber wo die Wergfasern ansonsten im Wesentlichen aneinander ungebunden sind,
**dadurch gekennzeichnet, dass** das Binden in zwei Schritten durchgeführt wird:
einem ersten Schritt, wobei das Binden durch Thermobonden, wie zum Beispiel Ultraschallschweißen oder Laserschweißen stattfindet, bei dem eine Musterrolle (22) verwendet wird, die das gewünschte Bindemuster bereitstellt, wobei die Musterrolle (22) mit einer höheren Geschwindigkeit angetrieben wird als der Zuführungsgeschwindigkeit der Werglage (15), um ein relativ diffuses und starkes Bindemuster (10a) in der Werglage (15) zu erzeugen;
und einem zweiten Schritt, in dem ein klareres zweites Bindemuster (10b) erzeugt wird.

2. Verfahren nach Anspruch 1, **dadurch gekennzeichnet, dass** das erste und zweite Bindemuster (10a, 10b) so aufgebracht werden, dass sie einander über zumindest einen wesentlichen Teil der gebundenen Fläche der Fasermateriallage überlappen.

3. Verfahren nach Anspruch 1 oder 2, **dadurch gekennzeichnet, dass** auch in dem zweiten Schritt das Binden durch Thermobonden, wie zum Beispiel Ultraschallschweißen oder Laserschweißen stattfindet, bei dem eine Musterrolle (25) verwendet wird, die das gewünschte Bindemuster bereitstellt.

4. Verfahren nach Anspruch 3, **dadurch gekennzeichnet, dass** die Musterrolle (25) in dem zweiten Bindeschritt im Wesentlichen mit derselben Geschwindigkeit wie der Zuführungsgeschwindigkeit der Werglage (15) angetrieben wird.

5. Verfahren nach Anspruch 3 oder 4, **dadurch gekennzeichnet, dass** die Geschwindigkeit der Musterrolle (22) des ersten Bindeschritts zumindest 8% höher als die Zuführungsgeschwindigkeit der Werglage (15) ist.

6. Verfahren nach Anspruch 5, **dadurch gekennzeichnet, dass** die Geschwindigkeit der Musterrolle (22) des ersten Bindeschritts zwischen 8 und 100% und bevorzugt zwischen 10 und 25% höher als die Zuführungsgeschwindigkeit der Werglage (15) ist.

7. Verfahren nach einem der Ansprüche 1 bis 6, **dadurch gekennzeichnet, dass** die Werglage (15) in dem zweiten Schritt an eine Materialbahn, wie zum Beispiel eine Vlieslage (26), laminiert wird.

8. Fasermateriallage, die hauptsächlich dafür gedacht ist, in einen Absorptionsartikel, wie zum Beispiel eine Windel, eine Höschenwindel, einen Inkontinenzschutz, eine Binde, einen Wundverband und dergleichen eingefügt zu werden, wobei die Materiallage eine Lage (15) aus durchgehenden Fasern (9), ein so genanntes Werg, umfasst, die in Punkten, Linien oder Flecken in einem Bindemuster (10) zusammengebunden sind, aber ansonsten im Wesentlichen aneinander ungebunden sind,
**dadurch gekennzeichnet, dass** die Werglage (15) mit zumindest zwei Bindemustern gebunden ist:
einem ersten, relativ starken und diffusen Bindemuster (10a), das durch Thermobonden bereitgestellt wird, und
einem zweiten klareren Bindemuster (10b);
wobei das erste und zweite Bindemuster so angeordnet ist, dass sie miteinander über zumindest einen wesentlichen Teil der gebundenen Fläche der Faserlage überlappen.

9. Fasermateriallage nach Anspruch 7, **dadurch gekennzeichnet, dass** auch in dem zweiten Bindemuster (10b) ein thermogebondetes Bindemuster ist.

10. Fasermateriallage nach Anspruch 8 oder 9, **dadurch gekennzeichnet, dass** eine Materialbahn, wie zum Beispiel eine Vlieslage (26) an die Werglage (15) mit dem zweiten Bindemuster (10b) laminiert ist.

11. Fasermateriallage nach einem der Ansprüche 8 bis 10, **dadurch gekennzeichnet, dass** zumindest ein Teil der durchgehenden Fasern (9) in der Werglage gequetscht oder gekräuselt ist.

12. Fasermateriallage nach einem der Ansprüche 8 bis 11, **dadurch gekennzeichnet, dass** das erste und zweite Bindemuster (10a, 10b) Punkte, Flecken oder Linien umfasst, welche die Längsrichtung der Wergfasern (9) kreuzen.

13. Faserlage nach Anspruch 12, **dadurch gekennzeichnet, dass** verschiedene Bindeorte, gesehen in der Querrichtung des Artikels, einander überlappen, so dass ein Hauptabschnitt der Wergfasern (9) zumindest an einem Teil ihrer Länge gebunden sind.

14. Faserlage nach Anspruch 12 oder 13, **dadurch gekennzeichnet, dass** individuelle Bindeorte der ersten und zweiten Bindemuster (10a, 10b) eine Längenerweiterung in einer Hauptrichtung, wie zum Beispiel Linien oder dergleichen, haben und dass die Bindelinien oder dergleichen des ersten Bindemusters (10a), verglichen mit den Bindelinien oder dergleichen des zweiten Bindemusters (10b), in einem verschiedenen Winkel zu der Hauptrichtung der Wergfasern (9) angeordnet sind.

15. Faserlage nach einem der Ansprüche 12 bis 14, **dadurch gekennzeichnet, dass** individuelle Bindeorte des ersten und zweiten Bindemusters (10a, 10b) eine Längenerweiterung in einer Hauptrichtung, wie zum Beispiel Linien oder dergleichen, haben und dass die Bindelinien oder dergleichen innerhalb des ersten und/oder des zweiten Bindemusters verschiedene Winkel in Bezug auf die Hauptrichtung der Fasern haben.

16. Absorptionsartikel, wie zum Beispiel eine Windel, Höschenwindel, ein Inkontinenzschutz, eine Binde, ein Wundverband und dergleichen, der Art, die eine flüssigkeitsdurchlässige Oberlage (2), eine flüssigkeitsundurchlässige Rücklage (3) und einen Absorptionskörper (4), der dazwischen angeordnet ist, umfassen, **dadurch gekennzeichnet, dass** der Artikel eine Werglage nach einem der Ansprüche 8 bis 15 umfasst.

17. Absorptionsartikel nach Anspruch 16, **dadurch gekennzeichnet, dass** die Werglage als eine Flüssigkeitserfassungslage (5) verwendet wird, die zwischen die Oberlage (2) und den Absorptionskörper (4) aufgelegt wird.

18. Absorptionsartikel nach Anspruch 16, **dadurch gekennzeichnet, dass** die Werglage als eine Flüssigkeitsdurchlässige Oberlage verwendet wird.

19. Absorptionsartikel nach einem der Ansprüche 16 bis 18, **dadurch gekennzeichnet, dass** die Werglage als eine integrierte Oberlage/Flüssigkeitserfassungslage verwendet wird.

## Revendications

1. Procédé de fabrication d'une couche de matériau fibreux destinée principalement à être incorporée dans un article absorbant tel qu'une couche, une couche d'apprentissage de la propreté, une protection contre l'incontinence, une serviette hygiénique, un pansement et des articles similaires, comprenant l'ouverture d'au moins un faisceau de fibres continues, appelé étoupe, la séparation des fibres de l'étoupe et l'aplanissement de l'étoupe jusqu'à former une couche (15) présentant la répartition désirée des fibres et en liaison de la couche d'étoupe selon un dessin de liaison (10) les autres fibres de l'étoupe étant substantiellement exemptes de liaison les unes avec les autres,
**caractérisé en ce que** la liaison est réalisée en deux étapes :
une première étape dans laquelle la liaison est effectuée par thermocollage, tel qu'un soudage par ultrasons ou un soudage par laser, dans laquelle est utilisé un rouleau (22) comportant un motif, lequel fournit le motif de liaison désiré, ledit rouleau (22) comportant un motif étant entraîné à une vitesse supérieure à la vitesse d'amenage de la couche d'étoupe (15), de manière à créer un motif de liaison (10a) relativement diffus et résistant dans la couche d'étoupe (15) ;
et une deuxième étape dans laquelle est créé un deuxième motif de liaison (10b) plus net.

2. Procédé selon la revendication 1,
**caractérisé en ce que** lesdits premier et deuxième motifs de liaison (10a, 10b) sont appliqués de manière à se chevaucher sur au moins une partie substantielle de la zone de liaison de ladite couche de matériau fibreux.

3. Procédé selon la revendication 1 ou 2,
**caractérisé en ce qu'**également dans la deuxième étape la liaison est effectuée par thermocollage tel que le soudage par ultrasons ou par laser, un rouleau (25) comportant un motif qui permet d'obtenir le motif de liaison désiré, étant utilisé.

4. Procédé selon la revendication 3,
**caractérisé en ce que** dans la deuxième étape de collage, le rouleau (25) comportant un motif est entraîné à substantiellement la même vitesse que la vitesse d'avance de la couche d'étoupe (15).

5. Procédé selon la revendication 3 ou 4,
**caractérisé en ce que** la vitesse du rouleau (22) comportant un motif, de la première étape de liaison, est supérieure d'au moins 8 % à la vitesse d'avance de la couche d'étoupe (15).

6. Procédé selon la revendication 5,
**caractérisé en ce que** la vitesse du rouleau (22) comportant un motif, de la première étape de liaison, est supérieure de 8 % à 10 %, et de préférence de 10 % à 25 % supérieure à la vitesse d'avance de la couche d'étoupe (15).

7. Procédé selon l'une quelconque des revendications 1 à 6,
**caractérisé en ce que** lors de la deuxième étape la couche d'étoupe (15) est stratifiée sur une bande de matériau, telle qu'une couche de non-tissé (26).

8. Couche de matériau fibreux destinée principalement à être incorporée dans un article absorbant, tel qu'une couche, une couche d'apprentissage de la propreté, une protection contre l'incontinence, une serviette hygiénique, un pansement et des articles similaires, ladite couche de matériau comprenant une couche (15) de fibres continues (9), appelée étoupe, qui ont été liées ensemble selon des points, des lignes ou des plages d'après un motif de liaison (10), mais qui ailleurs sont substantiellement non liées les unes avec les autres ;
**caractérisée en ce que** la couche d'étoupe (15) est liée selon au moins deux motifs de liaison :
un premier motif de liaison (10a) relativement résistant et diffus obtenu par thermocollage, et
un deuxième motif de liaison (10b) plus net ;
lesdits premier et deuxième motifs de liaison étant agencés de manière à se chevaucher sur au moins une partie substantielle de la surface liée de ladite couche de fibres.

9. Couche de matériau fibreux selon la revendication 7,
**caractérisée en ce que** le deuxième motif de liaison (10b) est également un motif de liaison thermocollé.

10. Couche de matériau fibreux selon la revendication 8 ou 9, **caractérisée en ce qu'**une bande de matériau, telle qu'une couche de non-tissé (26), est stratifiée sur la couche d'étoupe (15) au moyen dudit deuxième motif de liaison (10b).

11. Couche de fibres selon l'une quelconque des revendications 8 à 10, **caractérisée en ce qu'**au moins une partie des fibres continues (9) de ladite couche d'étoupe sont crêpées ou ondulées.

12. Couche de fibres selon l'une quelconque des revendications 8 à 11, **caractérisée en ce que** le premier et le deuxième motifs de liaison (10a, 10b) comprennent des points, des plages ou des lignes qui croisent la direction longitudinale des fibres (9) d'étoupe.

13. Couche de fibres selon la revendication 12,
**caractérisée en ce que** différents emplacements de liaison se chevauchent, vu dans la direction transversale de l'article, de telle manière qu'une partie principale des fibres (9) d'étoupe sont liées au moins sur une certaine partie de leur longueur.

14. Couche de fibres selon la revendication 12 ou 13,
**caractérisée en ce que** des emplacements de liaison individuels des premier et deuxième motifs de liaison (10a, 10b) s'étendent en longueur dans une direction principale, selon des lignes ou des formes similaires, et **en ce que** les lignes, ou les formes similaires, de liaison du premier motif de liaison (10a) sont agencées selon un angle différent par rapport à la direction principale des fibres (9) d'étoupe, en comparaison avec les lignes de liaison, ou les formes de liaison similaires, du deuxième motif de liaison (10b).

15. Couche de fibres selon l'une quelconque des revendications 12 à 14,
**caractérisée en ce que** des emplacements de liaison individuels des premier et deuxième motifs de liaison (10a, 10b) s'étendent en longueur dans une direction principale, selon des lignes ou des formes similaires, et **en ce que** les lignes de liaison ou les formes de liaison similaires du premier et/ou du deuxième motif présentent des angles différents par rapport à la direction principale des fibres.

16. Article absorbant tel qu'une couche, une couche d'apprentissage de la propreté, une protection contre l'incontinence, une serviette hygiénique, un pansement et des articles similaires, comprenant une feuille supérieure (2) perméable aux liquides, une feuille arrière (3) imperméable aux liquides et un corps absorbant (4) agencé entre celles-ci,
**caractérisé en ce que** l'article comprend une couche d'étoupe telle que revendiqué dans l'une quelconque des revendications 8 à 15.

17. Article absorbant selon la revendication 16,
**caractérisé en ce que** la couche d'étoupe est utilisée en tant que couche (5) destinée à recueillir des liquides, disposée entre la feuille supérieure (2) et le corps absorbant (4).

18. Article absorbant selon la revendication 16,
**caractérisé en ce que** la couche d'étoupe est utilisée en tant que feuille supérieure perméable aux liquides.

19. Article absorbant selon l'une quelconque des revendications 16 à 18,
**caractérisé en ce que** la couche d'étoupe est utilisée en tant que feuille supérieure/couche de recueil de liquides intégrées.
